Europäisches Patentamt

European Patent Office

Office européen des brevets

(11) Publication number : **0 517 515 A2**

# EUROPEAN PATENT APPLICATION

(21) Application number : **92305119.7**

(22) Date of filing : **04.06.92**

(51) Int. Cl.$^5$ : **C12N 15/10, C12N 1/06, B01D 61/00**

(30) Priority : **04.06.91 JP 159436/91**

(43) Date of publication of application :
**09.12.92 Bulletin 92/50**

(84) Designated Contracting States :
**CH DE FR GB LI SE**

(71) Applicant : **TOSOH CORPORATION**
**No. 4560, Kaisei-cho, Shinnanyo-shi**
**Yamaguchi-ken 746 (JP)**

(72) Inventor : **Ogawa, Kazuo**
**1-27-4, Aobacho, Higashimurayama-shi**
**Tokyo 189 (JP)**
Inventor : **Nishi, Akihiro**
**2398, Kawaraguchi, Ebina-shi**
**Kanagawa-ken 243-04 (JP)**

(74) Representative : **Kearney, Kevin David**
**Nicholas et al**
**KILBURN & STRODE 30 John Street**
**London, WC1N 2DD (GB)**

(54) **Method of purifying DNA.**

(57)    A method for purifying plasmid DNA and/or cosmid DNA is disclosed which can be carried out easily without time-consuming centrifugation and without using a toxic reagent. The method comprises lysing microorganism cells containing the plasmid DNA and/or cosmid DNA, filtering the resultant lysate through a membrane filter to obtain a filtrate, to thereby remove insoluble materials, and subjecting the filtrate to ultrafiltration to remove impurities and thereby condense the DNA.

Fig. 1

This invention relates to a method for purifying plasmid DNA and/or cosmid DNA. The method of the present invention is useful in the field of genetic engineering.

With the progress of genetic engineering techniques, nucleic acids are purified from various cells, viruses and phages. Especially, isolation and identification of plasmid DNAs from microorganisms such as E. coli are indispensable to genetic recombination techniques.

Purification of plasmid DNA is widely carried out by lysing a microorganism such as E. coli with an alkali, neutralizing and centrifuging the resultant, extracting the supernatant with phenol so as to remove proteins and by subjecting the resultant to ethanol precipitation so as to concentrate the DNA. Since the DNA solution obtained by this method contains RNAs and the like as impurities, as necessary, the DNA is further purified by treating the DNA solution with an RNase and by subjecting the resultant to polyethylene glycol precipitation. Alternatively, after the treatment with RNase, the DNA is purified by ultracentrifugation.

In the above-described conventional method, toxic reagents such as phenol and chloroform are used. Further, centrifugation is necessary in each step so that the process is time-consuming and troublesome Still further, in cases where an ultracentrifuge is used, in addition to the problem that the process is time-consuming, there is an economical problem that the ultracentrifuge is expensive.

Accordingly, one object of the present invention is to provide a method for purifying plasmid DNA and/or cosmid DNA by which the plasmid DNA and/or cosmid DNA may be purified to a high purity by simple operations without using a reagent toxic to humans.

The present invention stems from the finding that plasmid DNA and/or cosmid DNA may be purified to a high purity by lysing microorganism cells, filtering the lysate through a membrane filter (to remove insoluble materials) and by subjecting the filtrate to ultrafiltration (so as to remove impurities), thereby condensing the DNA.

That is, the present invention provides a method for purifying DNA, especially plasmid DNA and/or cosmid DNA, the method comprising, in the following order, lysing microorganism DNA-containing cells (for example, cells transfected or transformed with a vector, so that they preferably contain plasmid DNA and/or cosmid DNA); filtering the resultant (lysate) through a membrane filter to obtain a filtrate (such as to remove insoluble materials); and subjecting the filtrate to ultrafiltration (preferably to remove impurities), thereby condensing the DNA.

According to the method of the present invention, plasmid DNA and/or cosmid DNA may be purified to a high purity from microorganism cells by simple operations. Further, reagents toxic to humans need not be used. Further, since the method may have no centrifugation step, the time required for the purification of the DNA can be short.

The microorganism cells subjected to the method of the present invention may be any cells which contain a plasmid DNA and/or a cosmid DNA. For example, E. coli cells transformed with a plasmid such as pBR322, pUC18 or the like, cultured in 2 x YT medium containing ampicillin, which are collected by centrifuging the culture medium may be subjected to the method of the present invention.

In the first step of the method of the present invention, the microorganism cells containing a plasmid DNA and/or a cosmid DNA are lysed. The cells may be lysed by any conventional method. For example, the cells may be lysed by the treatment with aqueous alkaline metal hydroxide solution and/or with a surfactant. As the alkaline metal hydroxide, NaOH or KOH may preferably be employed at a concentration of, preferably, 0.01 - 1 N. As the surfactant, anionic surfactants such as sodium dodecyl sulfate (SDS) may preferably be employed at a concentration of, preferably, 0.1 - 1% by weight. Before this treatment, the cells may be treated with 1 - 10 mg of lysozyme.

When an alkaline metal hydroxide solution is used for lysing the cells, the lysate may be neutralized before being subjected to the second step. The neutralization may be carried out by adding an acid such as acetic acid or hydrochloric acid, or by adding a neutral buffer with a high concentration.

The cells may also be lysed by treating the cells with a surfactant under heat. As the surfactant used here, nonionic surfactants such as "Triton X-100" (trade name) may preferably be employed, at a concentration of, preferably, 0.1 - 1% by weight. The cells may be heated in a hot water bath at 90 - 100°C for 2 - 10 minutes.

In the second step, the cell lysate obtained in the above-described first step is filtered through a membrane filter. In view of the prevention of the insoluble materials from passing through the filter and of the filtration rate, the pore size of the membrane filter may preferably be 0.1 - 2 μm. The material constituting the membrane filter is not at all restricted and commercially available membrane filters may conveniently be used.

The filtrate obtained in the second step is then subjected to ultrafiltration. The ultrafiltration membrane used here preferably has a fractionation molecular weight of 30,000 to 1,000,000. If the fractionation molecular weight is smaller than 30,000, proteins may not pass through the membrane so that they may be contaminated in the obtained DNA as impurities. If the fractionation molecular weight is larger than 1,000,000, the desired DNA may pass through the membrane. The ultrafilter may be made of any material. For example, commercially available ultrafilters made of poysulfone may be employed. By the ultrafiltration, proteins and inorganic salts contained in the filtrate obtained in the second step are removed.

After the ultrafiltration, to increase the purity of the desired DNA, it is preferred to wash the ultrafilter by passing an appropriate solution through the ultrafilter. The solution for washing the ultrafilter is not restricted and widely used buffers such as TE buffer (Tris-HCl buffer containing EDTA) may be employed.

The purified DNA remaining on the ultrafilter may be recovered easily by washing the surface of the ultrafilter with an appropriate solution such as TE buffer.

Purified plasmid DNA and/or cosmid DNA may be obtained by the above-described steps. However, in some cases, RNAs contained in the cells may be contaminated in the desired DNA. In such cases, it is preferred to add a step of removing the RNAs. RNAs may be removed by using an RNase.

For example, a solution containing an RNase may be used for recovering the desired DNA from the ultrafilter so as to decompose the RNAs in the DNA solution. Alternatively, as the above-mentioned solution for washing the ultrafilter by passing the solution through the ultrafilter for removing impurities in the DNA remaining on the ultrafilter, a solution containing an RNase may be used. For example, a buffer containing an RNase may be used. If such a solution is used, RNAs are decomposed and then ultrafiltration is carried out, so that the decomposed RNAs are passed through the ultrafilter and so removed from the desired DNA on the ultrafilter. Still alternatively, as the solution for washing the ultrafilter by passing the solution through the ultrafilter, aqueous alkaline metal hydroxide solution may be employed. As the alkaline metal hydroxide, NaOH or KOH may be employed and the concentration thereof may preferably be 0.1 - 5N.

By washing the ultrafilter with the aqueous alkaline metal hydroxide solution, RNAs may be removed. After this treatment, if necessary, the ultrafilter may further be repeatedly washed by passing an appropriate solution such as TE buffer therethrough so as to adjust the pH of the DNA appropriately.

These treatments may be performed individually or in combination. By employing these steps, DNA with higher purity may be obtained.

The present invention will now be described by way of example with reference to the accompanying examples and drawing, in which:

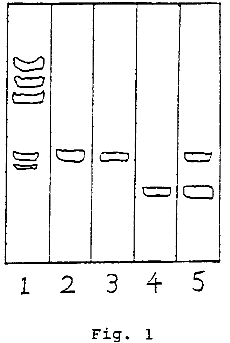

Fig. 1 shows agarose gel electrophoresis patterns of plasmid DNAs purified by the method of the present invention together with electrophoresis patterns of the commercially available plasmid DNAs.

It should be understood that the examples are presented for the purpose of illustration only and are not interpreted as being restrictive or limitative.

Example 1

E. coli JM105 transformed with plasmid pUC119 was cultured in 2 x YT medium containing ampicillin overnight at 37°C. Then 1.5 ml of the culture medium was centrifuged at 10,000 x g for 1 minute to collect the cells. The supernatant was discarded and the cells were suspended in 100 µl of 50 mM glucose/25mM Tris-HCl (pH8)/10 mM EDTA. After leaving the cell suspension to stand at 4°C for 5 minutes, 200 µl of 0.2 N NaoH/1 wt% sodium dodecyl sulfate (SDS) was added to the cell suspension. The resulting mixture was left to stand at 4°C for 5 minutes to lyse the cells. Then 150 µl of 3 M sodium acetate solution (pH 4.8) was added and the resultant was left to stand at this temperature for another 5 minutes.

The resulting mixture was filtered through a membrane filter having an average pore size of 0.22 µm under pressure. The filtrate was then filtered through an ultrafilter having a fractionation molecular weight of 100,000. After the ultrafiltration, the ultrafilter was washed by passing TE buffer therethrough. Then the ultrafilter was shaken with 100 µl of TE buffer containing 20 µg/ml of RNase, and the solution was then recovered by using a pipette.

Example 2

A culture medium of E. coli XL1-Blue transformed with plasmid pBluescript was subjected to lysing of the cells, filtration through the membrane filter and to ultrafiltration in the same manner as in Example 1. After the ultrafiltration, the ultrafilter was washed by passing 1 ml of 0.1 N NaOH solution therethrough. After further washing the ultrafilter by passing TE buffer therethrough, the DNA was recovered from the surface of the ultrafilter with 100 µl of TE buffer as in Example 1.

Example 3

The plasmid DNAs obtained in Examples 1 and 2 were analyzed by the conventional agarose gel electrophoresis. The results are shown in Fig. 1. For comparison, commercially available pUC119 and pBluescript plasmid DNAs were subjected to the same agarose gel electrophoresis. the results are also shown in Fig. 1. In Fig. 1, Lane 1 shows the electrophoresis pattern of Hind III digest of λ phage DNA, which is used as a molecular weight marker, Lanes 2 and 4 show the electrophoresis patterns of the commercially available plasmids pUC119 and pBluescript, respectively, and Lanes 3 and 5 show the electrophoresis patterns of the DNAs obtained in Examples 1 and 2, respectively.

As shown in Fig. 1, the DNAs purified by the method of the present invention had high purities.

**Claims**

1. A method of purifying plasmid DNA and/or cosmid DNA, the method comprising:
   lysing microorganism cells containing the plasmid DNA and/or cosmid DNA;
   filtering the resultant lysate through a membrane filter to remove any insoluble material to obtain a filtrate; and
   subjecting the filtrate to ultrafiltration to thereby condense the DNA.

2. A method according to Claim 1 wherein the pore size of the membrane filter is from 0.1 to 2 $\mu$m, and/or the molecular weight to be fractionated by ultrafiltration is from 30,000 to 1,000,000.

3. A method according to Claim 1 or 2 additionally comprising removing RNA originating from the microorganism cells.

4. A method according to Claim 3 wherein removal of RNA originating from the microorganism cells is achieved by recovering purified DNA remaining on the ultrafilter with a solution comprising an RNase.

5. A method according to Claim 3 wherein removal of RNA originating from the microorganism cells is achieved by washing the ultrafilter after ultrafiltration by passing a solution containing an RNase and/or an aqueous alkaline metal hydroxide through the ultrafilter.

6. A method according to any of Claims 1 to 5 wherein the cells are lysed by treatment with an aqueous alkaline hydroxide solution, such as NaOH and/or KOH, and/or a surfactant, such as SDS.

7. A method according to Claim 6 wherein the lysate is neutralised, such as with an acid, before filtration.

8. A method according to any of Claims 1 to 7 wherein the cosmid and/or plasmid DNA is derived from transfection or transformation of a cell with a vector.

9. A method according to any of Claims 1 to 8 wherein the cells comprise E. coli cells and/or the DNA to be purified is derived from plasmid pUC119 or pBluescript.

10. DNA purified by a method according to any of Claims 1 to 9.

Fig. 1